(19) Europäisches Patentamt / European Patent Office / Office européen des brevets

(11) EP 4 349 344 A1

(12) EUROPEAN PATENT APPLICATION
published in accordance with Art. 153(4) EPC

(43) Date of publication:
10.04.2024 Bulletin 2024/15

(21) Application number: 22811310.6

(22) Date of filing: 24.05.2022

(51) International Patent Classification (IPC):
A61K 33/26 (2006.01)    A61K 31/198 (2006.01)
A61K 31/405 (2006.01)    A61K 31/4172 (2006.01)
A23L 33/16 (2016.01)     A23L 33/175 (2016.01)
A61P 7/06 (2006.01)

(52) Cooperative Patent Classification (CPC):
A23L 33/16; A23L 33/175; A61K 31/198;
A61K 31/405; A61K 31/4172; A61K 33/26;
A61P 7/06

(86) International application number:
PCT/JP2022/021205

(87) International publication number:
WO 2022/250040 (01.12.2022 Gazette 2022/48)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 25.05.2021  JP 2021087813

(71) Applicant: AJINOMOTO CO., INC.
Chuo-ku
Tokyo 104-8315 (JP)

(72) Inventors:
• ICHIKAWA, Reiko
  Kawasaki-shi, Kanagawa 210-8681 (JP)
• SUSA, Yuko
  Kawasaki-shi, Kanagawa 210-8681 (JP)
• MURAKAMI, Hitoshi
  Kawasaki-shi, Kanagawa 210-8681 (JP)
• OHASHI, Haruka
  Kawasaki-shi, Kanagawa 210-8681 (JP)

(74) Representative: Strehl Schübel-Hopf & Partner
Maximilianstrasse 54
80538 München (DE)

(54) COMPOSITION FOR IMPROVING OR PREVENTING IRON DEFICIENCY ANAEMIA

(57) Compositions for improving or preventing iron deficiency anemia, that can be ingested easily and exhibit effects from an early stage, are provided. A composition for improving or preventing iron deficiency anemia, comprising methionine, threonine, and a pharmacologically acceptable iron compound as active ingredients.

[Fig. 2]

EP 4 349 344 A1

## Description

[Technical Field]

The present invention relates to compositions for improving or preventing iron deficiency anemia.

[Background Art]

Anemia is a condition in which the number of erythrocytes in the blood decreases or the concentration of hemoglobin contained in erythrocytes decreases. Hemoglobin plays a role in transporting oxygen from the lungs to various organs and tissues. As a result, headache, stiff neck, feeling of fatigue, dizziness, lightheadedness, tinnitus, palpitation, shortness of breath and the like occur as the symptoms of anemia. Among these, iron deficiency anemia occurs because hemoglobin production becomes insufficient due to lack of iron in the body.

Subjects who are more likely to develop iron deficiency anemia include children, women with menstruation, elderly people, athletes and the like. Particularly, about half of Japanese women in their 20s to 40s are said to suffer from "hidden iron deficiency" (latent iron deficiency). When iron from food is deficient, it is supplemented from stored iron in the body. In "hidden iron deficiency", shortage of stored iron continues, causing a decline in metabolic reactions that use iron as a cofactor (energy production and the like). If the shortage of stored iron continues, the hemoglobin concentration in the blood decreases, resulting in iron deficiency anemia. The problem is that feeling of fatigue, decreased endurance, and decreased sleep quality occur from the stage of "hidden iron deficiency".

Under these circumstances, there is a demand for a composition for improving or preventing iron deficiency anemia that can be ingested easily and exhibits effects from an early stage.

Regarding iron absorption from food, it has been reported that ascorbic acid, citric acid, and meat factor contained in meat promote the absorption of heme iron, and cysteine and cysteine-containing peptide promote the absorption of non-heme iron (Non Patent Literature 1).

[Citation List]

[Non Patent Literature]

[NPL 1]
Clinical Nutrition ESPEN, Volume 11, February 2016, Pages e1-e11

[Summary of Invention]

[Technical Problem]

The present invention aims to provide compositions for improving or preventing iron deficiency anemia, that can be ingested easily and exhibit effects from an early stage.

[Solution to Problem]

The present inventors have conducted intensive studies in an attempt to solve the aforementioned problems and confirmed that high-protein nutrition quickly improves iron deficiency in an iron-deficient rat model. They have conducted further studies and found that a specific combination of amino acids is effective in improving iron deficiency anemia, and completed the present invention.

That is, the present invention provides the following.

[1] A composition for improving or preventing iron deficiency anemia, comprising methionine, threonine, and a pharmacologically acceptable iron compound as active ingredients.
[2] The composition of [1], further comprising histidine and tryptophan.
[3] The composition of [1] or [2], further comprising ornithine.
[4] The composition of any one of [1] to [3], wherein the composition is for improving or preventing a decrease in serum iron concentration in iron deficiency anemia.
[5] The composition of any one of [1] to [3], wherein the composition is for improving or preventing a decrease in hemoglobin value in iron deficiency anemia.
[6] The composition of any one of [1] to [3], wherein the composition is for improving or preventing a symptom caused by iron deficiency anemia.

[7] The composition of any one of [1] to [6], wherein the composition is a pharmaceutical product.

[8] The composition of any one of [1] to [6], wherein the composition is a food.

[9] A method for improving or preventing iron deficiency anemia, comprising administering an effective amount of a composition comprising methionine, threonine, and a pharmacologically acceptable iron compound to a subject in need of improving or preventing iron deficiency anemia.

[10] The method of [9], wherein the composition further comprises histidine and tryptophan.

[11] The method of [9] or [10], wherein the composition further comprises ornithine.

[12] The method of any one of [9] to [11], wherein the method is for improving or preventing a decrease in serum iron concentration in iron deficiency anemia.

[13] The method of any one of [9] to [11], wherein the method is for improving or preventing a decrease in hemoglobin value in iron deficiency anemia.

[14] The method of any one of [9] to [11], wherein the method is for improving or preventing a symptom caused by iron deficiency anemia.

[15] Use of a composition comprising methionine, threonine, and a pharmacologically acceptable iron compound in the production of a composition for improving or preventing iron deficiency anemia.

[16] The use of [15], wherein the composition comprising methionine, threonine, and a pharmacologically acceptable iron compound further comprises histidine and tryptophan.

[17] The use of [15] or [16], wherein the composition comprising methionine, threonine, and a pharmacologically acceptable iron compound further comprises ornithine.

[18] The use of any one of [15] to [17], wherein the composition for improving or preventing iron deficiency anemia is a composition for improving or preventing a decrease in serum iron concentration in iron deficiency anemia.

[19] The use of any one of [15] to [17], wherein the composition for improving or preventing iron deficiency anemia is a composition for improving or preventing a decrease in hemoglobin value in iron deficiency anemia.

[20] The use of any one of [15] to [17], wherein the composition for improving or preventing iron deficiency anemia is a composition for improving or preventing a symptom caused by iron deficiency anemia.

[21] The use of any one of [15] to [20], wherein the composition for improving or preventing iron deficiency anemia is a pharmaceutical product.

[22] The use of any one of [15] to [20], wherein the composition for improving or preventing iron deficiency anemia is a food.

[Advantageous Effects of Invention]

According to the present invention, a composition for improving or preventing iron deficiency anemia can be provided. The composition of the present invention exhibits an early improving effect on iron deficiency anemia.

The composition of the present invention can be used for improving or preventing a decrease in serum iron concentration in iron deficiency anemia, improving or preventing a decrease in hemoglobin value in iron deficiency anemia, or improving or preventing symptoms (e.g., headache, stiff neck, feeling of fatigue, dizziness, lightheadedness, tinnitus, palpitation, shortness of breath and the like) caused by iron deficiency anemia.

The composition of the present invention is superior in safety since the active ingredient of the composition is an amino acid and there is little risk of side effects. It becomes possible to provide foods, supplements, pharmaceutical products, and the like that are suitable for oral ingestion and superior in safety.

[Brief Description of Drawings]

[Fig. 1]

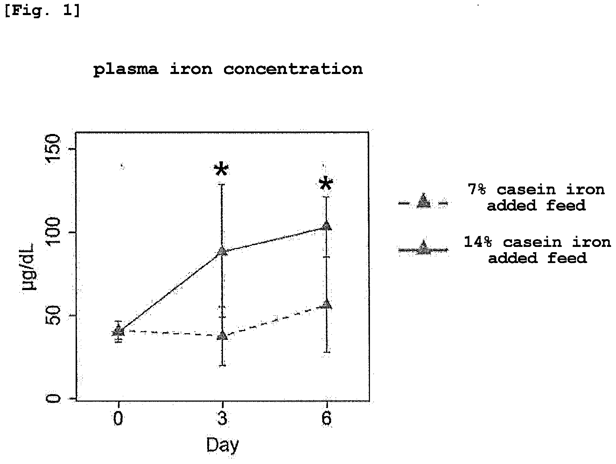

Fig. 1 is a graph showing the iron deficiency improving effect (plasma iron concentration) of protein nutrition, using iron deficient rats.

[Fig. 2]

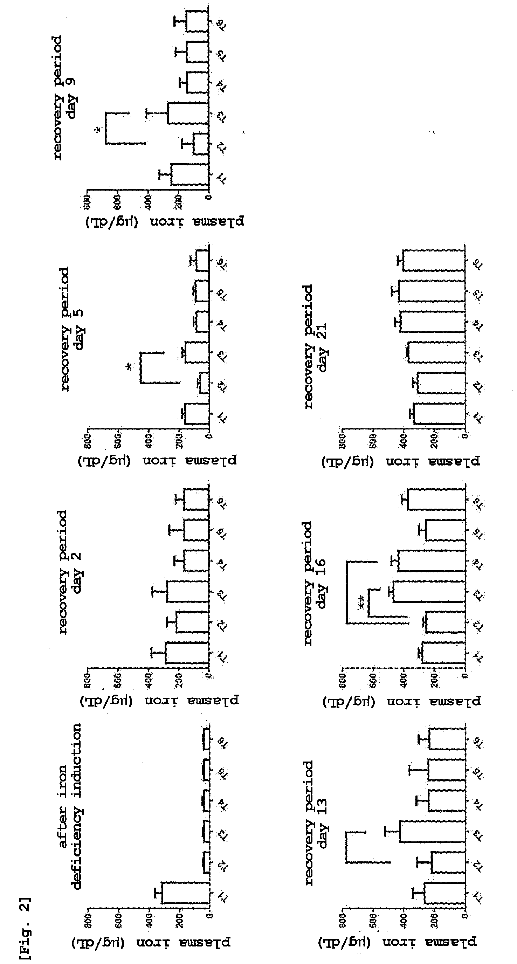

Fig. 2 is a graph showing the iron deficiency improving effect (plasma iron concentration) of the composition of the present invention in iron-deficient rats under low-protein nutrition.

[Fig. 3]

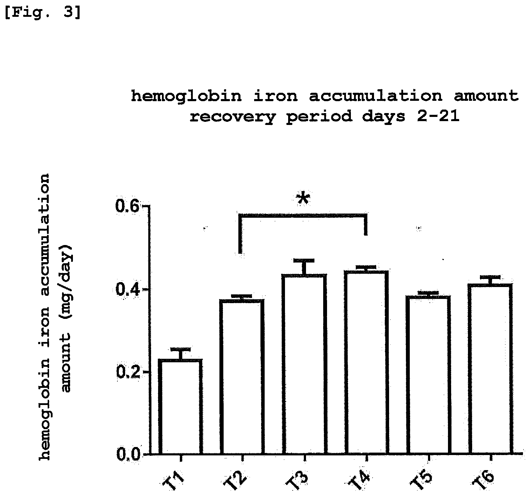

Fig. 3 is a graph showing the iron deficiency improving effect (hemoglobin iron accumulation amount) of the composition of the present invention in iron-deficient rats under low-protein nutrition.

[Description of Embodiments]

The composition for improving or preventing iron deficiency anemia of the present invention is a composition containing methionine, threonine, and a pharmacologically acceptable iron compound as active ingredients. The composition of the present invention may further contain histidine and tryptophan. Alternatively, the composition of the present invention may further contain ornithine.

In the present invention, "improving or preventing iron deficiency anemia" includes improving or preventing a decrease in serum iron concentration in iron deficiency anemia, improving or preventing a decrease in hemoglobin value in iron deficiency anemia, or improving or preventing a symptom caused by iron deficiency anemia. Examples of the symptom caused by iron deficiency anemia include headache, stiff neck, feeling of fatigue, dizziness, lightheadedness, tinnitus, palpitation, shortness of breath, hypodynamia, indefinite complaint (headache, irritation etc.), sleep insufficiency, decrease in intelligence/concentration, decrease in endurance, decrease in athletic performance, skin damage, deterioration of hair quality, broken nail, inflammation of lips, inflammation of tongue, and the like.

Furthermore, the composition of the present invention can provide the effects of increasing iron absorption and increasing iron availability. The composition of the present invention enables early recovery from iron deficiency and is useful for improving or preventing the aforementioned related symptoms that may be caused by iron deficiency.

. In the present invention, "iron deficiency anemia" includes latent iron deficiency called "hidden iron deficiency". The latent iron deficiency refers to a state in which indices such as serum iron concentration, serum ferritin value, and the like are lower than the normal range, even if the hemoglobin value, which is an index of anemia, is within the normal range.

The "pharmacologically acceptable iron compound" used in the present invention is not particularly limited as long as it is a non-toxic orally ingestible iron compound. For example, salts of iron with inorganic acids such as hydrochloric acid, sulfuric acid, phosphoric acid, pyrophosphoric acid, and the like (e.g., iron sulfate, ferric pyrophosphate); salts of iron with organic carboxylic acids such as citric acid, fumaric acid, maleic acid, succinic acid, tartaric acid, and the like (e.g., ferrous fumarate, sodium ferrous citrate, ferric citrate), and the like can be mentioned. The pharmacologically acceptable iron compound may be a hydrate (hydrate salt), and examples of such hydrate include 1 hydrate to 6 hydrate and the like.

Methionine, threonine, histidine, tryptophan, and ornithine, which are amino acids contained in the composition of the present invention, may be any of an L form, a D form and a DL form. An L form and a DL form are preferably used, and an L form is more preferably used.

The methionine, threonine, histidine, tryptophan, and ornithine can be used not only in a free form but also in a salt form. In the present invention, "free form" refers to a form in which a compound does not form a salt. The terms methionine, threonine, histidine, tryptophan, and ornithine in the present specification are each a concept also encompassing a salt. The salt form is not particularly limited as long as it is a pharmacologically acceptable salt, and an acid addition salt, salt with base and the like can be mentioned.

Concrete examples of the salt include salts with inorganic bases, salts with organic bases, salts with inorganic acids, salts with organic acids and the like.

Examples of the salts with inorganic bases include salts with alkali metals such as lithium, sodium, potassium and the like, salts with alkaline earth metals such as magnesium, calcium and the like, ammonium salt and the like.

Examples of the salts with organic bases include salts with an alkanolamine such as monoethanolamine, diethanolamine, triethanolamine and the like, salts with a heterocyclic amine such as morpholine, piperidine and the like, and the like.

Examples of the salts with inorganic acids include salts with a hydrohalic acid (hydrochloric acid, hydrobromic acid, hydroiodic acid etc.), sulfuric acid, nitric acid, phosphoric acid and the like.

Examples of the salts with organic acids include salts with a monocarboxylic acid such as formic acid, acetic acid, propanoic acid and the like; salts with a saturated dicarboxylic acid such as oxalic acid, malonic acid, malic acid, succinic acid and the like; salts with an unsaturated dicarboxylic acid such as maleic acid, fumaric acid and the like; salts with a tricarboxylic acid such as citric acid and the like; salts with a keto acid such as $\alpha$-ketoglutaric acid and the like.

The above-mentioned salts may each be a hydrate (hydrate salt), and examples of such hydrate include 1 hydrate to 6 hydrate and the like.

In the present invention, one kind each of methionine, threonine, histidine, tryptophan, and ornithine in the above-mentioned free form or salt form may be used singly, or two or more kinds thereof may be used in combination.

For the purpose of the present invention, methionine is preferably a free form, threonine is preferably a free form, histidine is preferably a free form, hydrochloride, or the like, tryptophan is preferably a free form, and ornithine is preferably a free form, hydrochloride, or the like.

In the present invention, the above-mentioned each amino acid in a free form or salt form to be used may be extracted from animals, plants or the like, which are naturally present, and purified, or obtained by a chemical synthesis method, a fermentation method, an enzyme method or a gene recombinant method and the like. Commercially available products provided by each company may also be utilized.

In the present specification, the blending ratio and content of each amino acid in the composition of the present invention when it is contained in a salt form are shown by the blending ratio and content converted to those of a free form.

In the composition of the present invention, the blending ratios of methionine, threonine, and the pharmacologically acceptable iron compound, each per 1 part by weight of methionine and when converted to a free form, are preferably 0.0001 to 1000 parts by weight for threonine and 0.0001 to 1 part by weight for the pharmacologically acceptable iron compound,

more preferably 0.003 to 300 parts by weight for threonine and 0.001 to 0.5 parts by weight (e.g., 0.001 to 0.1 part by weight) for the pharmacologically acceptable iron compound, and further preferably 0.025 to 40 parts by weight for threonine and 0.001 to 0.5 parts by weight (particularly preferably 0.001 to 0.05 parts by weight) for the pharmacologically acceptable iron compound.

When the composition of the present invention further contains histidine and tryptophan, the blending ratios of methionine, threonine, histidine, tryptophan, and the pharmacologically acceptable iron compound, each per 1 part by weight of methionine and when converted to a free form, are preferably 0.0001 to 1000 parts by weight for threonine, 0.0001 to 1000 parts by weight for histidine, 0.0001 to 1000 parts by weight for tryptophan, and 0.0001 to 1 part by weight for the pharmacologically acceptable iron compound,

more preferably 0.003 to 300 parts by weight for threonine, 0.003 to 300 parts by weight for histidine, 0.003 to 300 parts by weight for tryptophan, and 0.001 to 0.5 parts by weight (e.g., 0.001 to 0.1 part by weight) for the pharmacologically acceptable iron compound, and further preferably 0.025 to 40 parts by weight for threonine, 0.05 to 20 parts by weight for histidine, 0.05 to 20 parts by weight for tryptophan, and 0.001 to 0.5 parts by weight (particularly preferably 0.001 to 0.05 parts by weight) for the pharmacologically acceptable iron compound.

When the composition of the present invention further contains ornithine, the blending ratios of methionine, threonine, ornithine, and the pharmacologically acceptable iron compound, each per 1 part by weight of methionine and when converted to a free form, are

preferably 0.0001 to 1000 parts by weight for threonine, 0.0001 to 1000 parts by weight for ornithine, and 0.0001 to 1 part by weight for the pharmacologically acceptable iron compound, more preferably 0.003 to 300 parts by weight for threonine, 0.003 to 300 parts by weight for ornithine, and 0.001 to 0.5 parts by weight (e.g., 0.001 to 0.1 part by weight) for the pharmacologically acceptable iron compound, and further preferably 0.025 to 40 parts by weight for threonine, 0.05 to 20 parts by weight for ornithine, and 0.001 to 0.5 parts by weight (particularly preferably 0.001 to 0.05 parts by weight) for the pharmacologically acceptable iron compound.

The wt% of each amino acid in the composition of the present invention, with respect to the total amount of all amino acids in the composition and when converted to a free form is

preferably 0.5 to 30 wt% for methionine and 0.5 to 60 wt% for threonine, more preferably 1 to 15 wt% for methionine and 1.5 to 30 wt% for threonine, and further preferably 2 to 7 wt% for methionine and 3 to 12 wt% for threonine.

In another preferred embodiment, the wt% of each amino acid in the composition of the present invention, with respect to the total amount of all amino acids in the composition and when converted to a free form, is

preferably 0.1 to 99 wt% for methionine and 0.1 to 99 wt% for threonine, more preferably 1 to 50 wt% for methionine and 1 to 80 wt% for threonine, and further preferably 10 to 40 wt% for methionine and 30 to 70 wt% for threonine.

When the composition of the present invention further contains histidine and tryptophan, the wt% of each amino acid in the composition of the present invention, with respect to the total amount of all amino acids in the composition and when converted to a free form, is

preferably 0.5 to 30 wt% for methionine, 0.5 to 60 wt% for threonine, 0.5 to 30 wt% for histidine, and 0.2 to 10 wt% for tryptophan, more preferably 1 to 15 wt% for methionine, 1.5 to 30 wt% for threonine, 1 to 15 wt% for histidine, and 0.5 to 5 wt% for tryptophan, and

further preferably 2 to 7 wt% for methionine, 3 to 12 wt% for threonine, 2 to 6 wt% for histidine, and 1 to 3 wt% for tryptophan.

In another preferred embodiment, when the composition of the present invention further contains histidine and tryptophan, the wt% of each amino acid in the composition of the present invention, with respect to the total amount of all amino acids in the composition and when converted to a free form, is preferably 0.1 to 99 wt% for methionine, 0.1 to 99 wt% for threonine, 0.1 to 99 wt% for histidine, and 0.1 to 99 wt% for tryptophan,

more preferably 1 to 50 wt% for methionine, 1 to 80 wt% for threonine, 1 to 50 wt% for histidine, and 1 to 30 wt% for tryptophan, and
further preferably 10 to 40 wt% for methionine, 30 to 70 wt% for threonine, 10 to 30 wt% for histidine, and 5 to 15 wt% for tryptophan.

When the composition of the present invention further contains ornithine, the wt% of each amino acid in the composition of the present invention, with respect to the total amount of all amino acids in the composition and when converted to a free form, is

preferably 0.5 to 30 wt% for methionine, 0.5 to 60 wt% for threonine, and 0.5 to 30 wt% for ornithine,
more preferably 1 to 15 wt% for methionine, 1.5 to 30 wt% for threonine, and 1 to 15 wt% for ornithine, and
further preferably 2 to 7 wt% for methionine, 3 to 12 wt% for threonine, and 2 to 4 wt% for ornithine.

In another preferred embodiment, when the composition of the present invention further contains ornithine, the wt% of each amino acid in the composition of the present invention, with respect to the total amount of all amino acids in the composition and when converted to a free form, is preferably 0.1 to 99 wt% for methionine, 0.1 to 99 wt% for threonine, and 0.1 to 99 wt% for ornithine,

more preferably 1 to 50 wt% for methionine, 1 to 80 wt% for threonine, and 1 to 50 wt% for ornithine, and
further preferably 10 to 40 wt% for methionine, 30 to 70 wt% for threonine, and 10 to 30 wt% for ornithine.

The composition of the present invention may further contain, in addition to the above-mentioned amino acids, other nutrition components such as carbohydrates, lipid, protein, essential amino acid other than the above-mentioned amino acid, non-essential amino acid, vitamin, mineral and the like. In order to enhance iron absorption, the composition of the present invention may contain vitamin C.

The composition of the present invention can be formulated into various forms such as liquids (e.g., solution, suspension, emulsion and the like); semi-solid (e.g., gel, cream and the like); solid (e.g., powder, granule, tablet, capsule and the like), and the like by adding other nutrition components, pharmaceutically acceptable additives, and the like to the above-mentioned amino acids, as necessary and according to a formulating means well known in the field of preparations, for example, the methods described in the Japanese Pharmacopoeia 17th edition, General Rules for Preparations [3] Monographs for Preparations and the like. The composition of the present invention is preferably a composition for oral administration.

The above-mentioned pharmaceutically acceptable additive can be appropriately selected according to the form of the composition of the present invention and, for example, excipient, binder, disintegrant, lubricant, coating agent, base, solvent, solubilizing agents, solubilizer, emulsifier, dispersing agent, suspending agent, stabilizer, thickener, soothing agent, isotonicity agent, pH adjuster, antioxidant, antiseptic, preservative, corrigent, sweetener, flavor, colorant and the like can be mentioned.

To be specific, examples of the excipient include magnesium carbonate, saccharides (glucose, lactose, cornstarch etc.), sugar alcohol (sorbitol, mannitol etc.), silicon dioxide and the like.

Examples of the binder include gelatin, pregelatinized starch, partly pregelatinized starch, cellulose and a derivative thereof (crystalline cellulose, hydroxypropylcellulose etc.), dextrin and the like.

Examples of the disintegrant include crospovidone, povidone, crystalline cellulose and the like.

Examples of the lubricant include talc, magnesium stearate, calcium stearate and the like.

Examples of the coating agent include methacrylic acid-methyl methacrylate copolymer, methacrylic acid-ethyl acrylate copolymer, methyl methacrylate-butyl methacrylate-dimethylaminoethyl methacrylate copolymer, ethyl acrylate-methyl methacrylate-trimethylammonioethylmethacrylate chloride copolymer and the like.

Examples of the base include animal and plant fats and oils (olive oil, cacao butter, beef tallow, sesame oil, hydrogenated oil, castor oil etc.), wax (Carnauba wax, beeswax etc.), polyethylene glycol and the like.

Examples of the solvent include purified water, water for injection, monovalent alcohol (ethanol etc.), polyhydric alcohol (glycerol etc.) and the like.

Examples of the solubilizing agent include propylene glycol, medium-chain triglyceride and the like.

Examples of the solubilizer, emulsifier, dispersing agent and suspending agent include surfactant and the like such as sorbitan fatty acid ester, glycerol fatty acid ester, polyoxyethylene sorbitan fatty acid ester (polysorbate 20, polysorbate 80 etc.), polyoxyethylene hydrogenated castor oil, sucrose fatty acid ester and the like.

Examples of the stabilizer include adipic acid, β-cyclodextrin, ethylenediamine, sodium edetate and the like.

Examples of the thickener include water-soluble polymer (sodium polyacrylate, carboxyvinyl polymer etc.), polysaccharides (sodium alginate, xanthan gum, tragacanth etc.) and the like.

Examples of the soothing agent include ethyl aminobenzoate, chlorobutanol, propylene glycol, benzyl alcohol and the like.

Examples of the isotonicity agent include potassium chloride, sodium chloride, sorbitol, saline and the like.

Examples of the pH adjuster include hydrochloric acid, sulfuric acid, acetic acid, citric acid, lactic acid, sodium hydroxide, potassium hydroxide and the like.

Examples of the antioxidant include dibutylhydroxytoluene (BHT), butylhydroxyanisole (BHA), dl-α-tocopherol, erythorbic acid and the like.

Examples of the antiseptic and preservative include paraben (methylparaben etc.), benzyl alcohol, sodium dehydroacetate, sorbic acid and the like.

Examples of the corrigent include ascorbic acid, erythritol, sodium L-glutamate and the like.

Examples of the sweetener include aspartame, licorice extract, saccharin and the like.

Examples of the flavor include l-menthol, d-camphor, vanillin and the like.

Examples of the colorant include tar pigment (Food Color Red No. 2, Food Color Blue No. 1, Food Color Yellow No. 4 etc.), inorganic pigment (red iron oxide, yellow iron oxide, black iron oxide etc.), natural dye (turmeric extract, β-carotene, sodium copper-chlorophyllin etc.) and the like.

In the present invention, one or more kinds of the above-mentioned additives can be used.

The daily dose (ingestion amount) of the composition of the present invention is appropriately determined according to the sex, age of the subject to be applied to (hereinafter to be also referred to as the "application subject" in the present specification), conditions of iron deficiency anemia observed in the application subject and its degree, the form of the composition of the present invention, the administration method and the like.

Preferred ranges of the dose (ingestion amount) of the free amino acid contained in the composition of the present invention are shown in the following. In the present invention, the "free amino acid" refers to an amino acid not constituting a protein.

The dose (ingestion amount) of methionine per day for adult is preferably in the range of 1 mg to 10 g, more preferably in the range of 10 mg to 3 g, further preferably in the range of 50 mg to 1 g.

The dose (ingestion amount) of threonine per day for adult is preferably in the range of 1 mg to 10 g, more preferably in the range of 10 mg to 3 g, further preferably in the range of 50 mg to 2 g.

The dose (ingestion amount) of histidine per day for adult is preferably in the range of 1 mg to 10 g, more preferably in the range of 10 mg to 3 g, further preferably in the range of 50 mg to 1 g.

The dose (ingestion amount) of tryptophan per day for adult is preferably in the range of 1 mg to 10 g, more preferably in the range of 10 mg to 3 g, further preferably in the range of 50 mg to 1 g.

The dose (ingestion amount) of ornithine per day for adult is preferably in the range of 1 mg to 10 g, more preferably in the range of 10 mg to 3 g, further preferably in the range of 50 mg to 1 g.

The dose (ingestion amount) of the pharmacologically acceptable iron compound per day for adult and when converted to iron is preferably in the range of 0.1 mg to 40 mg, more preferably in the range of 1 mg to 30 mg, further preferably in the range of 3 mg to 20 mg.

The above-mentioned amount can be ingested or administered at once or in several portions (e.g., 2 to 4 portions) per day.

In addition, the ingestion or dosing period of the composition of the present invention is also appropriately determined according to the condition and symptoms of the application subject, and the like.

The composition of the present invention can be in the form of a package of a unit ingestion amount for one time or one meal, "unit package form". In such embodiment, the amount to be ingested once or per meal is determined in advance and packaged. The container or package used for the unit package form may be appropriately selected according to the form of the composition of the present invention and the like. Examples thereof include paper container or bag, plastic container or bag, pouch, aluminum can, steel can, bottle, plastic bottle, PTP (press through pack) packaging sheet and the like. In the case of drink, jelly, yogurt, gum, cookie or the like, for example, an amount to be ingested at one time is packaged in a container such as bag, pouch, bottle, box or the like. In the case of granule, powder, slurry or the like, for example, an amount to be ingested at one time is individually packaged in a pouch, bag or the like. Particularly, when the composition is a health food, food with functional claims, food with nutrient function claims, food for specified health uses and the like, for example, a form wherein the composition of the present invention is packed in a unit amount to be ingested once or per meal, a form wherein the composition of the present invention is suspended or dissolved to give a drink or a jelly, which is packaged in a pouch etc. for a single consumption or ingestion and the like can be mentioned.

When the composition of the present invention is in a unit package form for one time, an ingestion amount for one time in the unit preferably contains

methionine 1 mg to 10 g,
threonine 1 mg to 10 g, and
a pharmacologically acceptable iron compound when converted to iron 0.1 to 40 mg,
more preferably contains
methionine 50 mg to 1 g,
threonine 50 mg to 2 g, and
a pharmacologically acceptable iron compound when converted to iron 1 to 20 mg.

When the composition of the present invention further contains histidine and tryptophan and is in a unit package form for one time, an ingestion amount for one time in the unit preferably contains

methionine 1 mg to 10 g,
threonine 1 mg to 10 g,
histidine 1 mg to 10 g,
tryptophan 1 mg to 10 g, and
a pharmacologically acceptable iron compound when converted to iron 0.1 to 40 mg, and
more preferably contains
methionine 50 mg to 1 g,
threonine 50 mg to 2 g,
histidine 50 mg to 1 g,
tryptophan 100 mg to 1 g, and
a pharmacologically acceptable iron compound when converted to iron 1 to 20 mg.

When the composition of the present invention further contains ornithine and is in a unit package form for one time, an ingestion amount for one time in the unit preferably contains

methionine 1 mg to 10 g,
threonine 1 mg to 10 g,
ornithine 1 mg to 10 g, and
a pharmacologically acceptable iron compound when converted to iron 0.1 to 40 mg, and
more preferably contains
methionine 50 mg to 1 g,
threonine 50 mg to 2 g,
ornithine 50 mg to 1 g, and
a pharmacologically acceptable iron compound when converted to iron 1 to 20 mg.

The composition of the present invention can be applied to mammals (e.g., human, monkey, mouse, rat, guinea pig, hamster, rabbit, cat, dog, bovine, horse, donkey, swine, sheep etc.), birds (e.g., duck, chicken, goose, turkey etc.) and the like.

When the composition of the present invention is applied to an application target animal other than human (hereinafter to be also simply referred to as "target animal"), the ingestion amount or dose of the composition of the present invention can be appropriately set according to the kind, sex, body weight and the like of the target animal.

The composition of the present invention can be provided as it is or a pharmaceutical product further containing the above-mentioned pharmaceutically acceptable additives (hereinafter to be also referred to as "the pharmaceutical product of the present invention" in the present specification).

The pharmaceutical product of the present invention can have a dosage form of oral preparation such as tablet, coating tablet, chewable tablet, pill, (micro)capsule, granule, fine granule, powder, elixir, lemonade, syrup, suspension, emulsion, oral jelly and the like, injection such as solution, suspension, emulsion and the like, solid injection to be used by dissolving or suspending when in use, injectable preparation such as transfusion, sustainable injection and the like, tube feeding liquid and the like.

The pharmaceutical product of the present invention may contain other therapeutic drugs for iron deficiency anemia as long as the characteristics of the present invention are not impaired.

Furthermore, the composition of the present invention can be ingested by adding to various foods. The food to which the composition of the present invention is added is not particularly limited, and may be any as long as it is a food in the form generally served for meals and dessert.

For example, the composition of the present invention may be added to drinks (e.g., beverage etc.), and a suitable flavor is added when desired, whereby a drink can be provided.

More specifically, the composition of the present invention can be added to, for example, beverage water such as fruit juice drinks, sport drinks and the like; daily product such as cow milk, yogurt and the like; confectionery such as jelly, chocolate, candy, biscuit and the like, and the like.

The composition of the present invention is preferably added to the above-mentioned various foods in amounts to be ingested per day such that the amounts of each amino acid and pharmacologically acceptable iron compound in the present invention would be the above-mentioned ingestion amount per day.

The composition of the present invention can be provided as it is or as a food by adding general food additives where necessary by a general food production technique (hereinafter to be also referred to as "food of the present invention" in the present specification).

The food of the present invention can be prepared as various forms such as liquid (e.g., solution, suspension, emulsified liquid and the like); semi-solid (e.g., gel, cream and the like); solid (e.g., powder, granule, sheet, capsule, tablet and the like), and the like.

Furthermore, the food of the present invention can be prepared as various food forms such as beverage water (fruit juice drinks, sport drinks, coffee drinks, tea drinks etc.), beverage mixed with milk, dairy product (lactic fermenting beverage, fermented milk, butter, cheese, yogurt, processed milk, defatted milk etc.), meat product (ham, sausage, hamburger steak etc.), fish meat processed seafood paste product (fish cake, tube-shaped fish sausage, deep-fried ball of fish paste etc.), egg product (rolled Japanese-style egg omelette, steamed egg custard etc.), confectionery (cookie, jelly, custard pudding, chocolate, cake, chewing gum, candy, snack food, frozen dessert etc.), bread, noodles, pickle, dried fish, food boiled in soy sauce, soup, rice porridge, nutrition bar, batter for fried food, dressing, seasoning and the like by adding the composition of the present invention to various food starting materials and adding general food additives as necessary. It may also be a bottled food, canned food or retort pouch food.

In addition, the composition of the present invention can be provided as a feed or a pet food.

As the above-mentioned food additive, manufacturing agent (brine, binding agent etc.), thickening stabilizer (xanthan gum, sodium carboxymethylcellulose etc.), gelation agent (gelatin, agar, carrageenan etc.), gum base (vinyl acetate resin, jelutong, chicle etc.), emulsifier (glycerol fatty acid ester, sucrose fatty acid ester, saponin, lecithin etc.), preservative (benzoic acid, sodium benzoate, sorbic acid, potassium sorbate, $\varepsilon$-polylysine etc.), antioxidant (ascorbic acid, erythorbic acid, catechin etc.), glazing agent (shellac, paraffin wax, beeswax etc.), fungicide (thiabendazole, fludioxonil etc.), leavening agent (sodium hydrogen carbonate, glucono-$\delta$-lactone, alum etc.), sweetener (aspartame, acesulfame potassium, licorice extract etc.), bittering agent (caffeine, naringin, worm wood extract etc.), acidulant (citric acid, tartaric acid, lactic acid etc.), seasoning (sodium L-glutamate, disodium 5'-inosinate etc.), colorant (annatto dye, turmeric dye, gardenia dye etc.), flavor (synthetic flavor such as ethyl acetoacetate, anisaldehyde and the like, natural flavor such as orange, lavender and the like) and the like can be mentioned.

In the present invention, one or more kinds of the above-mentioned food additive can be used.

Therefore, the food of the present invention can also be provided as food with health claims such as a food for specified health uses, food with nutrient function claims, food with functional claims and the like, food for special dietary uses such as food for sick people, food for the elderly and the like, health supplement and the like for the improvement or prevention of iron deficiency anemia.

The food of the present invention is preferably given to the above-mentioned application subject such that the amounts of each amino acid and pharmacologically acceptable iron compound in the present invention would be the above-mentioned ingestion amount per day.

[Examples]

The present invention is explained in more detail in the following by referring to Examples. The present invention is not limited to the following Examples. In the present specification, unless otherwise specified, % means wt%.

Example 1

Verification of iron deficiency improving effect of protein nutrition using iron-deficient rats

Materials and methods

[1. Animals]

Female, 3-week-old Crlj:WI rats purchased from Charles River Laboratories Japan, Inc. were used.

[2. Feeds for acclimatization, iron deficiency induction, and recovery]

Feeds for acclimatization, iron deficiency induction, and iron deficiency recovery in rats were produced. The basic composition was AIN-93G, and a casein content of the feed to be given to the low-protein group was 7%. The decrease in casein was adjusted by the amount of corn starch. Each composition is as shown in Table 1.

[Table 1]

| | for acclimatization (14%CA+Fe) | for iron deficiency induction (7%CA-Fe) | for recovery (low-protein) (7%CA+Fe) | for recovery (high-protein) (14%CA+Fe) (g) |
|---|---|---|---|---|
| vitamin-free casein (Oriental Yeast Co., Ltd.) | 140 | 70 | 70 | 140 |
| L-cystine (Ajinomoto Co., Inc.) | 1.8 | 1.8 | 1.8 | 1.8 |
| cornstarch (Nisshoku) | 465.6 | 535.7 | 535.6 | 465.6 |
| α-starch (Oriental Yeast Co., Ltd.) | 155 | 155 | 155 | 155 |
| powder sugar (sucrose) | 100 | 100 | 100 | 100 |
| cellulose (Toyo Roshi Kaisha, Ltd.) | 50 | 50 | 50 | 50 |
| soybean oil | 40 | 40 | 40 | 40 |
| t-butylhydroquinone (FUJIFILM Corporation) | 0.008 | 0.008 | 0.008 | 0.008 |
| mineral mix (Oriental Yeast Co., Ltd.) | 35 | 35 | 35 | 35 |
| iron (III) citrate n-hydrate (FUJIFILM Corporation) | 0.07 | - | 0.07 | 0.07 |
| AIN93 vitamin mix (Oriental Yeast Co., Ltd.) | 10 | 10 | 10 | 10 |
| choline hydrogen tartrate (FUJIFILM Corporation) | 2.5 | 2.5 | 2.5 | 2.5 |
| **Total (g)** | **1000** | **1000** | **1000** | **1000** |

CA: casein, Fe: iron(III) citrate n-hydrate

[Blood iron concentration measurement]

Blood was collected from the rat tail vein using a hematocrit capillary tube, plasma was separated by centrifugation, and the plasma iron concentration was measured using a kit.

[Method]

The rats were 3 weeks old when delivered and acclimatized using acclimatization feed for 1 week. Thereafter, iron deficiency was induced by allowing free ingestion of iron deficiency induction feed for 3 weeks. Thereafter, the rats were divided into a low-protein diet group (casein 7%) or a high-protein diet group (casein 14%) supplemented with iron (n=5/group), and 20 g per animal was given per day. Blood samples were collected after iron deficiency induction (Day 0), on 3rd day (Day 3) and 6th day (Day 6) of recovery, and plasma iron concentration was measured.

[Results]

Significant recovery of plasma iron concentration was observed in rats fed with the high-protein iron deficiency recovery feed (Fig. 1). *:$p<0.05$, Dunnett's test with Day 0

Example 2

Verification of iron deficiency improving effect of amino acid mix under low-protein nutrition using iron-deficient rats Materials and methods

[1. Animals]

Female, 3-week-old Crlj:WI rats purchased from Charles River Laboratories Japan, Inc. were used.

[2. Feeds for acclimatization, iron deficiency induction, and recovery]

Feeds for acclimatization, iron deficiency induction, and iron deficiency recovery in rats were produced. The basic composition was AIN-93G, and a casein content was 7%. The composition of each amino acid mix feed is shown in Table 2.

[Table 2]

| | for acclimatization (14%CA+ Fe) | for iron deficiency induction (7%CA-Fe) | T1, T2 for recovery (low-protein) (7%CA+Fe) | T3 for recovery (low-protein) (7%CA+Fe) +Met,Thr | T4 for recovery (low-protein) (7%CA+Fe) +Met,Thr, Trp,His | T5 for recovery (low-protein) (7%CA+Fe) +Cys2, Thr | T6 for recovery (low-protein) (7%CA+Fe) +Met, Thr, Orn |
|---|---|---|---|---|---|---|---|
| | | | | | | | (g) |
| vitamin-free casein (Oriental Yeast Co., Ltd.) | 140 | 70 | 70 | 70 | 70 | 70 | 70 |
| L-cystine (Ajinomoto Co., Inc.) | 1.8 | 0.9 | 0.9 | 0.9 | 0.9 | 0.9 | 0.9 |
| cornstarch (Nisshoku) | 465.5 | 536.6 | 536.4 | 529.4 | 529.3 | 529.4 | 529.4 |
| $\alpha$-starch (Oriental Yeast Co., Ltd.) | 155 | 155 | 155 | 155 | 155 | 155 | 155 |
| powder sugar (sucrose) | 100 | 100 | 100 | 100 | 100 | 100 | 100 |
| cellulose (Toyo Roshi Kaisha, Ltd.) | 50 | 50 | 50 | 50 | 50 | 50 | 50 |
| soybean oil | 40 | 40 | 40 | 40 | 40 | 40 | 40 |
| t-butylhydroquinone (FUJIFILM Corporation) | 0.008 | 0.008 | 0.008 | 0.008 | 0.008 | 0.008 | 0.008 |
| mineral mix (Oriental Yeast Co., Ltd.) | 35 | 35 | 35 | 35 | 35 | 35 | 35 |
| iron(III) citrate n-hydrate (FUJIFILM Corporation) | 0.212 | - | 0.15 | 0.15 | 0.15 | 0.15 | 0.15 |
| AIN93 vitamin mix (Oriental Yeast Co., Ltd.) | 10 | 10 | 10 | 10 | 10 | 10 | 10 |
| choline hydrogen tartrate (FUJIFILM Corporation) | 2.5 | 2.5 | 2.5 | 2.5 | 2.5 | 2.5 | 2.5 |

EP 4 349 344 A1

(continued)

| | for acclimatization (14%CA+ Fe) | for iron deficiency induction (7%CA-Fe) | T1, T2 for recovery (low-protein) (7%CA+Fe) | T3 for recovery (low-protein) (7%CA+Fe) +Met,Thr | T4 for recovery (low-protein) (7%CA+Fe) +Met,Thr, Trp,His | T5 for recovery (low-protein) (7%CA+Fe) +Cys2, Thr | T6 for recovery (low-protein) (7%CA+Fe) +Met, Thr, Orn (g) |
|---|---|---|---|---|---|---|---|
| L-methionine (Ajinomoto Co., Inc.) | - | - | - | 2.3 | 1.6 | - | 1.6 |
| L-cystine (Ajinomoto Co., Inc.) | - | - | - | - | - | 2.3 | - |
| L-threonine (Ajinomoto Co., Inc.) | - | - | - | 4.7 | 3.1 | 4.7 | 3.1 |
| L-tryptophan (Ajinomoto Co., Inc.) | - | - | - | - | 0.8 | - | - |
| L-histidine (Ajinomoto Co., Inc.) | - | - | - | - | 1.6 | - | - |
| L-(+)-ornithine monohydrochloride (Nacalai Tesque, Inc.) | - | - | - | - | - | - | 2.3 |
| total (g) | 1000 | 1000 | 1000 | 1000 | 1000 | 1000 | 1000 |

CA: casein, Fe: iron(III) citrate n-hydrate, Met: methionine, Thr: threonine, Trp: tryptophan, His: histidine, Cys2: cystine, Orn: ornithine

[Hemoglobin concentration measurement and hemoglobin iron accumulation amount calculation]

Blood samples were collected from the rat tail vein using a hematocrit capillary tube, and the blood hemoglobin concentration was measured using FUJI DRI-CHEM 7000 (FUJIFILM Medical Co., Ltd.). In addition, the amount of iron used in hemoglobin synthesized during recovery from iron deficiency was calculated using the following formula.

```
Hb-Fe =
((Wt1*dHb)+(dWt*dHb)+(dWt*dHb))*0.075(L/Kg)*10(d)*3.39(mg/g)/10
00(k)*dDays
```

Hb-Fe: hemoglobin iron accumulation amount (mg/day)
Wt1: body weight (g) immediately before recovery from iron deficiency
dHb: hemoglobin increase amount (g/dL) before and after recovery from iron deficiency
dWt: body weight gain (g) before and after recovery from iron deficiency
dDays: iron recovery period (day)
0.075(L/Kg): Blood amount per kilogram of body weight (L/Kg)

[Plasma iron concentration measurement]

Blood was collected from the rat tail vein using a hematocrit capillary tube, plasma was separated by centrifugation, and the plasma iron concentration was measured using a kit.

[Method]

The rats were 3 weeks old when delivered and acclimatized using acclimatization feed for 1 week. Iron deficiency was induced by allowing free ingestion of iron deficiency induction feed for 4 weeks. Thereafter, the feed was changed to each iron deficiency recovery feed and the rats were subjected to the test. The iron deficiency recovery feed was provided in an equal amount per rat body weight. The feed provided to each group is shown in Table 3.

[Table 3]

| group | iron deficiency induction period | iron deficiency recovery period | n number |
|---|---|---|---|
| T1 | 7%CA+Fe | 7%CA+Fe | 7 |
| T2 | 7%CA-Fe | 7%CA+Fe | 7 |
| T3 | 7%CA-Fe | 7%CA+Fe+Met+Thr | 7 |
| T4 | 7%CA-Fe | 7%CA+Fe+Met+Thr+Trp+His | 7 |
| T5 | 7%CA-Fe | 7%CA+Fe+Cys2+Thr | 7 |
| T6 | 7%CA-Fe | 7%CA+Fe+Met+Thr+Orn | 7 |

Blood samples were collected the day immediately before (Day -1), 2 days after (Day 2), 5 days after (Day 5), 9 days after (Day 9), 13 days after (Day 13), 16 days after (Day 16), and 21 days after (Day 21) provision of the iron deficiency recovery feed and plasma iron concentration was measured. In addition, hemoglobin concentration was measured the day before (Day -1) and 21 days after (Day 21) provision of the iron deficiency recovery feed, and hemoglobin iron accumulation amount was calculated.

[Results]

Plasma iron concentration recovered 2 days after changing to the iron deficiency recovery feed, and remained significantly higher in the T3 group that ingested methionine+threonine (Met+Thr) blend feed from days 5 to 13. On day 16, a significant increase in plasma iron was observed in T3 group and also in T4 group that ingested methionine+threonine+tryptophan+histidine (Met+Thr+Trp+His) blend feed. Furthermore, although not significant, the mean plasma iron concentration remained higher after 16 days from changing in the T6 group to which methionine+threonine+ornithine (Met+Thr+Orn) blend feed was given, as compared with the control T2 group (Fig. 2). Dunn's multiple comparison test *:p<0.05, **:p<0.01, vs T2 group

In the comparison of hemoglobin iron accumulation, which is an index of hemoglobin synthesis amount, a significant increase was observed in the methionine+threonine+tryptophan+histidine (Met+Thr+Trp+His) T4 group as compared with the control group (T2 group), and an increasing tendency was also observed in the methionine+threonine (Met+Thr) T3 group (Fig. 3). Dunn's multiple comparison test *:p<0.05, vs T2 group

Example 3

A composition of the present invention (food or composition for oral administration) having the formulation shown in Table 4 was prepared by a conventional method.

[Table 4]

| general name | mixing ratio (%) | per one meal (mg) |
| --- | --- | --- |
| L-methionine | 27.03 | 200 |
| L-threonine | 54.05 | 400 |
| sodium ferrous citrate | 7.80 | 57.69 (6.12 mg when converted to iron) |
| cellulose | 7.62 | 56.40 |
| silicon dioxide | 1.50 | 11.10 |
| calcium stearate | 2.00 | 14.80 |
| total | 100 | 740 |

[Industrial Applicability]

According to the present invention, it is possible to provide a composition for improving or preventing iron deficiency anemia.

This application is based on a patent application No. 2021-087813 filed in Japan (filing date: May 25, 2021), the contents of which are incorporated in full herein by reference.

**Claims**

1. A composition for improving or preventing iron deficiency anemia, comprising methionine, threonine, and a pharmacologically acceptable iron compound as active ingredients.

2. The composition according to claim 1, further comprising histidine and tryptophan.

3. The composition according to claim 1, further comprising ornithine.

4. The composition according to any one of claims 1 to 3, wherein the composition is for improving or preventing a decrease in serum iron concentration in iron deficiency anemia.

5. The composition according to any one of claims 1 to 3, wherein the composition is for improving or preventing a decrease in hemoglobin value in iron deficiency anemia.

6. The composition according to any one of claims 1 to 3, wherein the composition is for improving or preventing a symptom caused by iron deficiency anemia.

7. The composition according to any one of claims 1 to 3, wherein the composition is a pharmaceutical product.

8. The composition according to any one of claims 1 to 3, wherein the composition is a food.

9. A method for improving or preventing iron deficiency anemia, comprising administering an effective amount of a composition comprising methionine, threonine, and a pharmacologically acceptable iron compound to a subject in need of improving or preventing iron deficiency anemia.

10. The method according to claim 9, wherein the composition further comprises histidine and tryptophan.

11. The method according to claim 9, wherein the composition further comprises ornithine.

12. The method according to any one of claims 9 to 11, wherein the method is for improving or preventing a decrease in serum iron concentration in iron deficiency anemia.

13. The method according to any one of claims 9 to 11, wherein the method is for improving or preventing a decrease in hemoglobin value in iron deficiency anemia.

14. The method according to any one of claims 9 to 11, wherein the method is for improving or preventing a symptom caused by iron deficiency anemia.

15. Use of a composition comprising methionine, threonine, and a pharmacologically acceptable iron compound in the production of a composition for improving or preventing iron deficiency anemia.

16. The use according to claim 15, wherein the composition comprising methionine, threonine, and a pharmacologically acceptable iron compound further comprises histidine and tryptophan.

17. The use according to claim 15, wherein the composition comprising methionine, threonine, and a pharmacologically acceptable iron compound further comprises ornithine.

18. The use according to any one of claims 15 to 17, wherein the composition for improving or preventing iron deficiency anemia is a composition for improving or preventing a decrease in serum iron concentration in iron deficiency anemia.

19. The use according to any one of claims 15 to 17, wherein the composition for improving or preventing iron deficiency anemia is a composition for improving or preventing a decrease in hemoglobin value in iron deficiency anemia.

20. The use according to any one of claims 15 to 17, wherein the composition for improving or preventing iron deficiency anemia is a composition for improving or preventing a symptom caused by iron deficiency anemia.

21. The use according to any one of claims 15 to 17, wherein the composition for improving or preventing iron deficiency anemia is a pharmaceutical product.

22. The use according to any one of claims 15 to 17, wherein the composition for improving or preventing iron deficiency anemia is a food.

[Fig. 1]

plasma iron concentration

[Fig. 2]

[Fig. 3]

hemoglobin iron accumulation amount
recovery period days 2-21

## INTERNATIONAL SEARCH REPORT

| International application No. |
|---|
| **PCT/JP2022/021205** |

**A. CLASSIFICATION OF SUBJECT MATTER**

*A61K 33/26*(2006.01)i; *A61K 31/198*(2006.01)i; *A61K 31/405*(2006.01)i; *A61K 31/4172*(2006.01)i; *A23L 33/16*(2016.01)i; *A23L 33/175*(2016.01)i; *A61P 7/06*(2006.01)i

FI: A61K33/26; A61P7/06; A61K31/4172; A61K31/405; A23L33/175; A23L33/16; A61K31/198

According to International Patent Classification (IPC) or to both national classification and IPC

**B. FIELDS SEARCHED**

Minimum documentation searched (classification system followed by classification symbols)

A61K33/26; A61K31/198; A61K31/405; A61K31/4172; A23L33/16; A23L33/175; A61P7/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | CN 112409202 A (HENAN JIUTIAN BIOTECHNOLOGY CO., LTD.) 26 February 2021 (2021-02-26) claims, paragraph [0007], examples (in particular, example 3) | 1, 3-9, 11-15, 17-22 |
| Y | claims, paragraph [0007], examples (in particular, example 3) | 2, 10, 16 |
| Y | WO 2007/029730 A1 (MEIJI DAIRIES CORPORATION) 15 March 2007 (2007-03-15) paragraph [0010] | 2, 10, 16 |
| Y | WO 2009/066562 A1 (MEIJI DAIRIES CORPORATION) 28 May 2009 (2009-05-28) paragraph [0033] | 2, 10, 16 |
| X | JP 2008-50277 A (AJINOMOTO CO INC) 06 March 2008 (2008-03-06) claims, examples (test group (vii)) | 1, 2, 4-10, 12-16, 18-22 |
| A | JP 7-53391 A (SNOW BRAND MILK PROD CO LTD) 28 February 1995 (1995-02-28) entire text | 1-22 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **20 June 2022** | **19 July 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2022/021205**

| Patent document cited in search report | | | Publication date (day/month/year) | Patent family member(s) | | | Publication date (day/month/year) |
|---|---|---|---|---|---|---|---|
| CN | 112409202 | A | 26 February 2021 | (Family: none) | | | |
| WO | 2007/029730 | A1 | 15 March 2007 | (Family: none) | | | |
| WO | 2009/066562 | A1 | 28 May 2009 | CN | 101868230 | A | |
| JP | 2008-50277 | A | 06 March 2008 | (Family: none) | | | |
| JP | 7-53391 | A | 28 February 1995 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021087813 A

**Non-patent literature cited in the description**

- *Clinical Nutrition ESPEN,* February 2016, vol. 11, e1-e11